# EUROPÄISCHE PATENTSCHRIFT

(11) **EP 2 373 261 B1**
(45) Veröffentlichungstag und Bekanntmachung des Hinweises auf die Patenterteilung: **02.10.2013**
(21) Anmeldenummer: 09805678.1
(22) Anmeldetag: 11.12.2009
(51) Int. Cl.: A61F 2/30, A61F 2/36, A61F 2/46

(54) **VORRICHTUNG ZUM BESTIMMEN DER PASSFÄHIGKEIT ZWISCHEN EINEM ADAPTER UND EINEM AUSSENKONUS EINES PROTHESENSCHAFTES EINER MODULAREN GELENKPROTHESE**
DEVICE FOR DETERMINING THE COMPATIBILITY BETWEEN AN ADAPTER AND AN OUTER CONE OF A PROSTHESIS SHAFT OF A MODULAR JOINT PROSTHESIS
DISPOSITIF POUR DÉTERMINER LA COMPATIBILITÉ ENTRE UN ADAPTATEUR ET LE CÔNE EXTÉRIEUR DE LA TIGE D'UNE PROTHÈSE ARTICULAIRE MODULAIRE

(30) Priorität: 18.12.2008 DE 102008062730; 08.12.2009 DE 102009057117
(43) Veröffentlichungstag der Anmeldung: 12.10.2011
(73) Patentinhaber: Merete Medical GmbH, 12247 Berlin (DE)
(72) Erfinder: ANAPLIOTIS, Emmanuel, 14195 Berlin (DE); KRANZ, Curt, 14163 Berlin (DE); HILSE, Martin, 12105 Berlin (DE)
(74) Vertreter: Hannig, Wolf-Dieter
(86) Internationale Anmeldenummer: PCT/DE2009/001740
(87) Internationale Veröffentlichungsnummer: WO 2010/069292

(56) Entgegenhaltungen:
- WO-A1-2005/089676
- DE-A1- 10 335 442
- US-A1- 2006 217 815

## Beschreibung

Die Erfindung betrifft eine Vorrichtung zum Bestimmen der Passfähigkeit zwischen einem Adapter zum Einsatz in eine zum Austausch vorgesehene Gelenkkugel fremder Bauart und einen Außenkonus eines im Femur eingesetzten Prothesenschafts einer modularen Gelenkprothese während einer Revisionsoperation.

Es ist bekannt, dass es eine Vielzahl von im Einsatz befindlichen modularen Gelenkprothesensystemen unterschiedlicher Hersteller gibt, die insbesondere beim Austausch beschädigter oder verschlissener Komponenten einer Gelenkprothese, wie Gelenkkugel oder Adapter zu Problemen bei der Auswahl der richtigen prothetischen Komponente während der Operation führen. Oftmals ist dies dadurch bedingt, dass der Hersteller die Produktion der entsprechenden Gelenkprothese eingestellt hat oder auch der Hersteller nicht mehr am Markt tätig ist. Der Chirurg muss während des Eingriffs in kurzer Zeit entscheiden, ob ein Adapter oder eine Gelenkkugel eines anderen Herstellers zum Außenkonus des in den Femur eingesetzten Prothesenschaftes passt oder ein vollkommen neues modulares Gelenkprothesensystem eingesetzt werden muss.
Letztere Vorgehensweise verlängert für den Patienten den schon verhältnismäßig lange andauernden Eingriff und ist vielfach mit der Durchtrennung des Femur verbunden (US 5 100 407 A). Auch die Auswahl eines auf den Außenkonus des femoralen Prothesenschaftes passenden Adapters aus einem Satz von Adaptern nach dem Trial- und Error-Verfahren ist langwierig und für den Patienten letztendlich unbefriedigend (EP 0 767 638 B1).
Darüber hinaus ist aus der DE 10329 241 A1 ein Prüfinstrument zur Beurteilung des Konussitzes von Hüftendoprothesen mittels einer Prüffolie bekannt, die es ermöglicht den Traganteil eines Konussitzes festzustellen.

Weiterhin ist aus der US 2006/0217815A1 ein Satz von verschiedenen Köpfen für eine Hüftprothese mit unterschiedlichen konischen Ausnehmungen zum Aufsetzen auf einen Prothesenschaft bekannt.

### Aufgabenstellung

Bei diesem Stand der Technik liegt der Erfindung die Aufgabe zugrunde, ein Verfahren und eine Vorrichtung zur Bestimmung der Passfähigkeit eines Adapters Gelenkprothese anzugeben, die es ermöglichen, modulare Komponenten einer Gelenkprothese unterschiedlicher Hersteller miteinander zu kombinieren, die Revisionsoperation zu vereinfachen und Gelenkkugeln aus Keramik oder Metall auf einem bereits liegenden Schaft passgenau einzusetzen.

Diese Aufgabe wird durch eine Vorrichtung der eingangs genannten Gattung mit den Merkmalen der Ansprüche 1 gelöst.

Vorteilhafte Ausgestaltungen der erfindungsgemäßen Vorrichtung sind den Unteransprüchen entnehmbar.

Die erfindungsgemäße Lösung zeichnet sich dadurch aus, dass die Komponenten von modularen Gelenkprothesen unterschiedlicher Hersteller durch den Einsatz eines entsprechend angepassten Adapters miteinander kompatibel und kombinierbar werden. Dies ist mit dem außerordentlichen Vorteil für den Patienten verbunden, dass der im Femur eingesetzte Prothesenschaft nicht entfernt und nur ein an die vorliegenden geometrischen Verhältnisse des Außenkonus des Prothesenschaftes angepasster Adapter eingesetzt werden muss, um die Kompatibilität zwischen den neuen und den vorhandenen Komponenten der Prothese herzustellen.

Die Kombination der Komponenten von modularen Gelenkprothesen unterschiedlicher Hersteller gelingt durch eine Bestimmung der Passfähigkeit zwischen einem Innenkonus eines Adapters und einem Außenkonus des Schaftes einer Gelenkprothese, wobei eine Markierung und Längen-Messskala aufweisender, visuell einsehbarer Prüfkörper in Form eines Satzes von Prüfkörpern mit unterschiedlichen Innenkonen auf den Schaft der Gelenkprothese sukzessive aufgesteckt und anschließend der auf den Schaft der Gelenkprothese passende Adapter durch ein Testen der Innenkonen auf Einhaltung eines zulässig minimalen Abstandes zwischen der Stirnseite des Außenkonus des Schaftes der Gelenkprothese und dem Innenkonus sowie einer als zulässig vorgegebenen Einstecktiefe des Außenkonus der Gelenkprothese in den Innenkonus des Prüfkörpers ermittelt wird.

Die erfindungsmäße Lösung gewährleistet, dass der Chirurg den zum Einsatz geeigneten Adapter entsprechend den tatsächlich vorhandenen geometrischen Verhältnissen am Außenkonus des Schaftes der Gelenkprothese mit folgenden Teilschritten sicher auswählen kann:
a) Feststellen des tatsächlichen Abstandes zwischen Stirnseite des Außenkonus des Schaftes der Gelenkprothese und dem Dach des Innenkonus des Prüfkörpers,
b) Vergleich des tatsächlichen Abstandes gemäß Schritt a) mit der Markierung am Prüfkörper als Maß für den minimal zulässigen Abstand zwischen Stirnseite des Außenkonus des Schaftes der Gelenkprothese und dem Dach des Innenkonus des Prüfkörpers,
c) Abziehen des aufgesetzten Prüfkörpers vom Außenkonus des Schaftes der Gelenkprothesen sobald im Schritt b) ermittelt wird, dass der zulässige Abstand unterschritten wird und Aufstecken eines weiteren Prüfkörpers aus dem Satz der Prüfkörper mit einem anderen Innenkonus,
d) Fortführen der Schritte a) bis c) solange bis der tatsächlich festgestellte Abstand mit der Markierung exakt übereinstimmt oder diese überschreitet,
e) Beurteilen der Rotationsfestigkeit und der Kippneigung durch Drehen des Prüfkörpers auf dem Außenkonus und bei Feststellen eines spürbaren Gleitreibungswiderstandes ohne Kippneigung,
f) Ermitteln der tatsächlichen Einstecktiefe des Außenkonus des Schaftes der Gelenkprothese in den Innenkonus des Prüfkörpers als ein Maß für den sicheren Sitz des Innenkonus auf dem Außenkonus des Schaftes der Gelenkprothese durch ein Ablesen der Längen-Messskala am Prüfkörper,
g) Ausschließen des gewählten Innenkonus des Prüfkörpers sobald im Schritt f) ermittelt wird, dass die tatsächliche Einstecktiefe die minimal zulässige Einstecktiefe unterschreitet und Abziehen des Prüfkörpers,
h) Aufstecken eines weiteren Prüfkörpers und Fortführen der Schritte a) bis d) solange bis die tatsächlich ermittelte Einstecktiefe größer ist als die minimal zulässige Einstecktiefe unter Einhaltung des minimal zulässigen Abstands,
i) Auswahl eines bevorrateten Adapters mit dem gemäß Schritt h) ermittelten Innenkonus zum Einsetzen in die Gelenkkugel.

Von besonderem Vorteil ist weiterhin, dass das Maß des minimal zulässigen Abstands aus den Toleranzen der entsprechenden Durchmesser und Winkel des Innenkonus der Prüfkörper und des Außenkonus des Schaftes der Gelenkprothesen bestimmt werden, so dass es möglich wird, geometrisch ungeeignete Adapter sicher und schnell auszuschließen und ein Wackeln und eine Rotation des Adapters auf dem Außenkonus des Schaftes der Gelenkprothese zu verhindern.

Es hat sich gezeigt, dass das Maß für die minimal zulässige Einstecktiefe des Außenkonus des Schaftes der Gelenkprothese in den Innenkonus des Prüfkörpers von der Länge der Überdeckung der beiden Konen abhängt und ausreichend ist, wenn die Einstecktiefe mindestens 7 mm beträgt. Es können Prüfkörper in Form eines angeschnittenen Kugelsegments oder eines Zylinders verwendet werden.

Die erfindungsgemäße Vorrichtung hat einen einfachen und überschaubaren Aufbau, der besonders für schnelle und sichere Bestimmung des richtigen Adapters geeignet ist.
Die Vorrichtung umfasst einen Satz Prüfkörper mit jeweils unterschiedlichen Innenkonen, wobei eine Markierung zum Feststellen des minimal zulässigen Abstands zwischen einer Stirnseite des Außenkonus des Schaftes der Gelenkprothese und einem Dach des Innenkonus des Prüfkörpers nahe des Daches an einer Schnittkante des Innenkonus vorgesehen ist, und dass an der Schnittkante des Innenkonus eine Längenmessskala zum Ermitteln der Einstecktiefe des Außenkonus des Schaftes in den Innenkonus des jeweiligen Prüfkörpers angeordnet ist.
Mit diesen beiden Messmitteln kann der Chirurg während der Operation schnell den minimal zulässigen Abstand zwischen der Stirnseite des Außenkonus des Schaftes der Gelenkprothese und die Einstecktiefe des Außenkonus des Schaftes in den Innenkonus des Prüfkörper visuell ermitteln und sofort eine Entscheidung treffen, ob der ausgewählte Innenkonus zur Anpassung geeignet oder ungeeignet ist.
Der Prüfkörper kann die Form eines von außen visuell einsehbaren Kugelsegments oder eines angeschnittenen Zylinders aufweisen.

Weitere Vorteile und Einzelheiten ergeben sich aus der nachfolgenden Beschreibung unter Bezugnahme auf die beigefügten Zeichnungen.

### Ausführungsbeispiel

Die Erfindung soll nachstehend an einem Ausführungsbeispiel näher erläutert werden.
Es zeigt

Fig. 1 eine Vorderansicht des Prüfkörpers der erfindungsgemäßen Vorrichtung in Form eines angeschnittenen Kugelsegments mit Markierung zur Bestimmung des minimal zulässigen Abstand und Längen-Messskala zur Ermittlung der Einstecktiefe,

Fig. 2 eine Seitenansicht gemäß Fig. 1,

Fig. 3 eine schematische Ansicht der erfindungsgemäßen Vorrichtung mit Kugelsegment im auf den Außenkonus des Schaftes der Gelenkprothese aufgesteckten Zustand zur Bestimmung des minimal zulässigen Abstand,

Fig. 4 eine schematische der erfindungsgemäßen Vorrichtung mit Kugelsegment im auf den Außenkonus des Schaftes der Gelenkprothese aufgesteckten Zustand zur Bestimmung der Einstecktiefe,

Fig. 5 eine schematische Ansicht des Prüfkörpers der erfindungsgemäßen Vorrichtung in Form eines angeschnittenen Zylinders und

Fig. 6 eine schematische Darstellung der Vorgehensweise zum Bestimmen der Passfähigkeit.

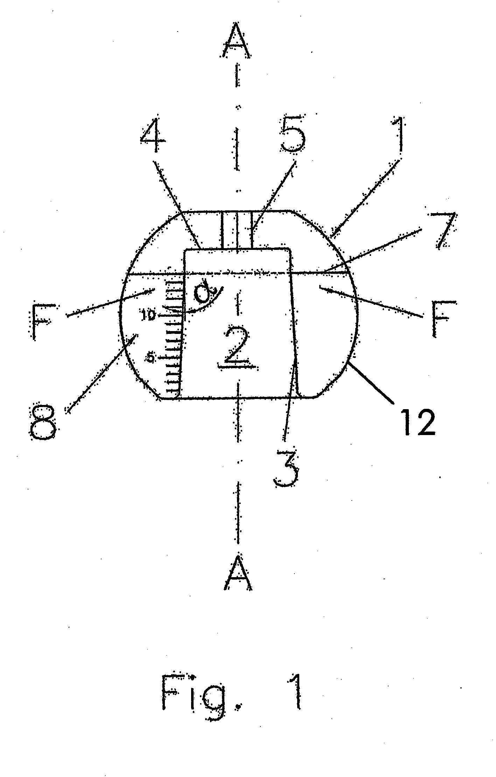

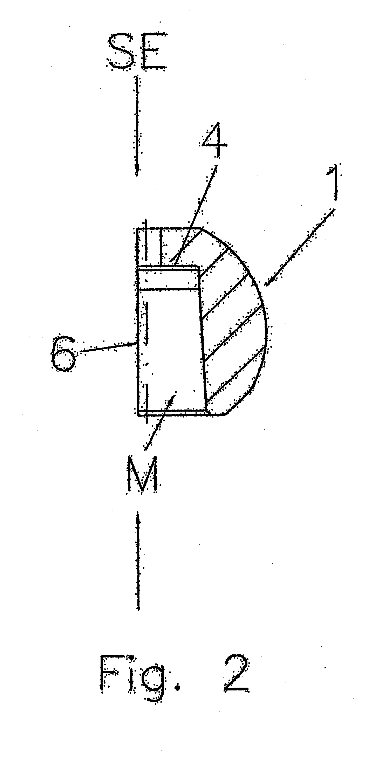

Die Fig. 1 zeigt den grundsätzlichen Aufbau der erfindungsgemäßen Vorrichtung zum Bestimmen der Passfähigkeit zwischen einem Adapter zum Einsatz in eine zum Austausch vorgesehene Gelenkkugel fremder Bauart und einen Außenkonus eines im Femur eingesetzten Prothesenschafts einer modularen Gelenkprothese.
Die erfindungsgemäße Vorrichtung umfasst wenigstens einen Prüfkörper 1 mit einem Messraum 2, der von einem mantelflächig geöffneten Innenkonus 3 und einem Dach 4 begrenzt ist. Das Dach 4 bildet den oberen Abschluss des Meßraumes 2. In das Dach 4 ist zentrisch eine in den Meßraum 2 führende Bohrung 5 eingebracht, die zur Aufnahme bzw. Einsetzen eines hier nicht weiter beschriebenen Trenninstruments dient. Der Prüfkörper 1 kann kugelsegmentartig (siehe Fig. 1 bis 4) oder auch zylinderartig ausgebildet sein.
Der Innenkonus 2 hat in seinem Mantel M eine Öffnung 6, die durch einen Schnitt entlang einer Ebene SE des kugelsegmentartig ausgebildeten Prüfköpers 1 entsteht, wodurch die Öffnung 6 durch parallel Achse A des Prüfkörpers 1 verlaufende Flächen F begrenzt ist (siehe Fig. 2). In diese Flächen F ist nahe dem Dach 4 eine Markierung 7 und eine Längen-Messskala 8 eingebracht, wobei die Markierung 7 zugleich Ausgangspunkt der Längen-Messskala 8 ist. Die Öffnung 6 ist dabei so positioniert, dass sowohl die Markierung 7, und die Längen-Messskala 8 durch den Chirurgen ablesbar und die Stirnseite 9 des Außenkonus 10 des Schaftes 11 der Gelenkprothese erkennbar ist.

Zu einer erfindungsgemäßen Vorrichtung gehört wenigstens ein Satz von Prüfkörpern 1 mit unterschiedlich von Prüfkörper zu Prüfkörper abgestuften Konuswinkeln α und Durchmessern D.

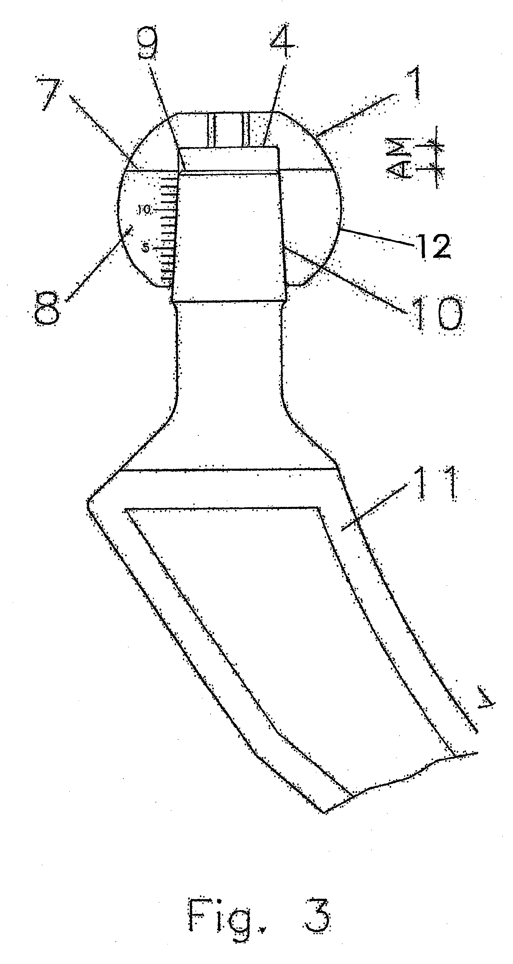

Die Fig. 3 zeigt den Prüfkörper 1 im auf den Außenkonus 10 eines Schaftes 11 einer Gelenkprothese aufgesteckten Zustand zur Bestimmung des Abstandes AM zwischen der Stirnseite 9 des Außenkonus 10 und dem Dach 4 des Prüfkörpers 1. In dem gezeigten Beispiel stimmt die Markierung 7 fast mit der Lage der Stirnseite 9 des Außenkonus 10 des Schaftes 11 der Gelenkprothese überein. Die Lage der Markierung 7 auf der Fläche F ergibt sich für jeden Prüfkörper 1 individuell aus den bekannten Toleranzen der entsprechenden Durchmesser und Winkel des Innenkonus 3 des Prüfkörpers 1 und des Außenkonus 10 des Schaftes 11 der Gelenkprothese sowie eines vorzugebenden Sicherheitsfaktors. Der minimal zulässige Abstand AM ist dann eingehalten, wenn zwischen der Lage der Markierung 7 und der Stirnseite 9 des Außenkonus 10 Übereinstimmung besteht. Ist der Abstand AM zu gering, kann es zum Aufliegen der Flächen kommen, wodurch ein Wackeln, eine Rotation und Verschleiß nicht mehr ausgeschlossen werden können. Das Fehlen der Übereinstimmung zwischen Markierung 7 und der Lage der Stirnseite 9 zeigt daher an, dass der Abstand AM zu gering und daher zum Ausschluss des gewählten Innenkonus 3 führt.

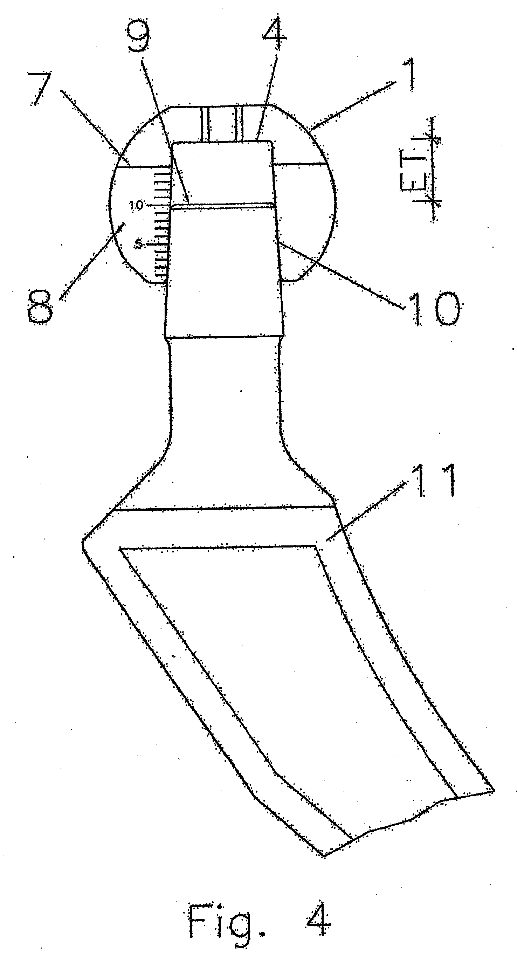

Die Fig. 4 zeigt die Bestimmung der Einstecktiefe ET des Außenkonus 10 in den Innenkonus 3 des Prüfkörpers 1. Die Einstecktiefe ET bestimmt den sicheren Halt des Innenkonus 3 auf dem Außenkonus 10 und hängt im Wesentlichen von der Länge der Überdeckung der beiden Konenflächen ab. Es hat sich gezeigt, dass ein sicherer Halt dann gewährleistet ist, wenn die Einstecktiefe ET mindestens 7 mm beträgt.

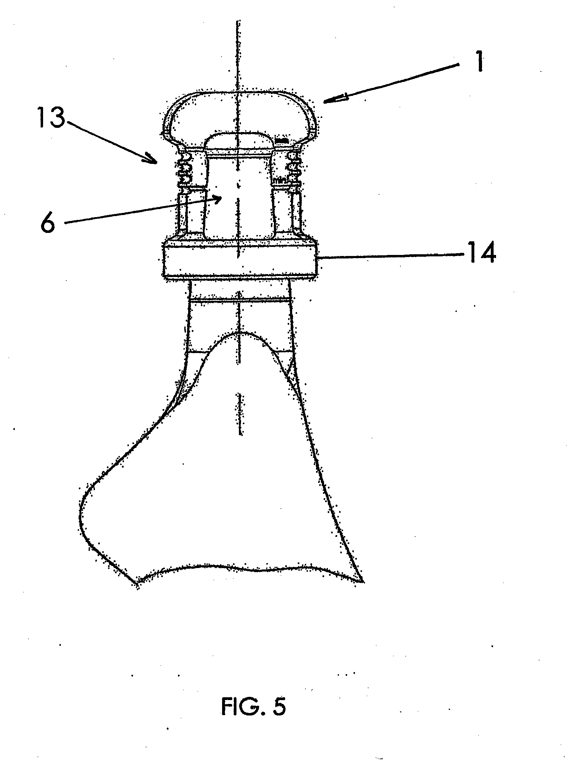

Die Fig. 5 zeigt eine Variante des Prüfkörpers 1 in Form eines angeschnittenen Zylinders 13, der unterhalb seiner Öffnung 6 mit einem umlaufenden Bund 14 versehen ist. Die Öffnung 6 im Mantel M des Prüfkörpers 1 entspricht dem im Abschnitt [0020] beschriebenen grundsätzlichen Aufbau. Die Längen-Messskala 8 besitzt hier Markierungen für einen minimalen und einen maximalen Ablesepunkt.

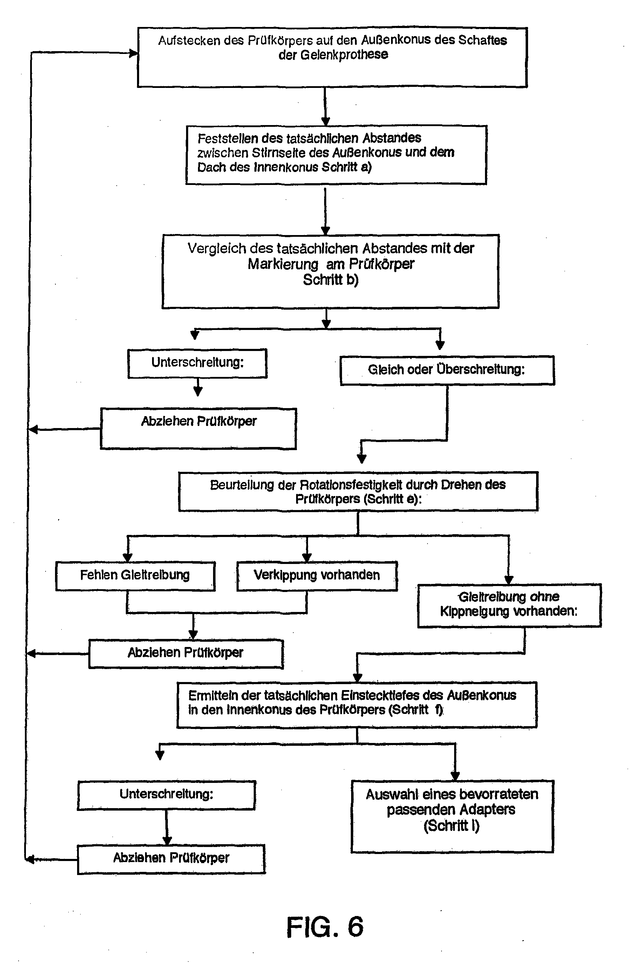

Die Fig. 6 zeigt schematisch die Vorgehensweise zum Bestimmen der Passfähigkeit.
Der Prüfkörper 1 eines Satzes von Prüfkörpern mit jeweils unterschiedlichen Innenkonen 2 wird auf den Außenkonus 10 des Schaftes 10 der Gelenkprothese aufgesteckt und der tatsächliche Abstand AM zwischen der Stirnseite 9 des Außenkonus 10 und dem Dach 4 des Innenkonus 3 ermittelt (Schritt a). Dazu wird die Lage der Stirnseite 9 mit der Markierung 7 auf der Fläche F des Prüfkörpers 1 verglichen. Ergibt der Vergleich, dass eine Unterschreitung des Abstandes AM vorliegt, wird der Prüfkörper 1 vom Außenkonus 10 des Schaftes 11 abgezogen und ein neuer Prüfkörper 1 aus dem Satz von Prüfkörpern mit einem beispielsweise größeren Konusdurchmesser D bzw. einem größeren Konuswinkel α ausgewählt. Dieser neue Prüfkörper 1 wird dann wiederum auf den Außenkonus 10 des Schaftes 11 der Gelenkprothese aufgesteckt. Es folgt die Ermittlung des Abstandes AM. Wird festgestellt, dass der Abstand AM eingehalten oder durch den tatsächlichen Abstand überschritten ist, erfolgt eine Beurteilung der Rotationsfestigkeit und des Kippneigung des Prüfkörpers 1 auf dem Außenkonus 10 des Schaftes 11 (Schritt e) durch Drehen des Prüfkörpers 1 auf dem Außenkonus 10. Sofern keine Kippneigung festgestellt wird und eine spürbare Gleitreibung beim Drehen zu überwinden ist, erfolgt das Ermitteln der tatsächlichen Einstecktiefe ET des Außenkonus 10 des Schaftes 11 in den Innenkonus 3 des Prüfkörpers 1 (Schritt f). Ist keine spürbare Gleitreibung ohne Kippneigung vorhanden, wird der Prüfkörper 1 abgezogen und ein neuer Prüfkörper 1 aus dem Satz der Prüfkörpers mit wiederum veränderten Konusdurchmesser D und Konuswinkel α auf den Außenkonus 10 aufgesteckt. Der Prüfvorgang beginnt erneut mit Schritt a).
Wird bei der Ermittlung der Einstecktiefe ET festgestellt, dass die tatsächliche Einstecktiefe einen in Abhängigkeit der Konuslänge vorgebene minimal zulässige Einstecktiefe überschreitet, liegen die für die Auswahl einzuhaltenden Bedingungen für die Passfähigkeit des Innenkonus 3 des vorliegenden Prüfkörpers vor und der entsprechend passende Adapter für die Gelenkkomponente kann aus einem dafür bereitgehaltenen Vorrat ausgewählt werden.

Die Erfindung beschränkt sich in ihrer Ausführung nicht auf das vorgenannte Ausführungsbeispiele Vielmehr sind Varianten denkbar, welche von der dargestellten Lösung auch bei grundsätzlich anders gearteten Ausführungen abweichen können.

Bezugszeichenliste

| | |
|---|---|
| Prüfkörper | 1 |
| Messraum | 2 |
| Innenkonus | 3 |
| Dach | 4 |
| Bohrung | 5 |
| Öffnung | 6 |
| Markierung | 7 |
| Längen-Messskala | 8 |
| Stirnseite | 9 |
| Außenkonus | 10 |
| Schaft | 11 |
| Kugelsegment | 12 |
| Zylinder | 13 |
| Umlaufender Bund | 14 |
| Achse von 1 | A |
| Abstand Stirnseite-Dach | AM |
| Durchmesser Konus | D |
| Einstecktiefe | ET |
| Fläche von 1 | F |
| Mantel von 3 | M |
| Ebene | SE |
| Konuswinkel | α |

Hierzu 5 Blatt Zeichnungen

## Patentansprüche

1. Vorrichtung zum Bestimmen der Passfähigkeit zwischen einem Adapter zum Einsatz in eine zum Austausch vorgesehene Gelenkkugel fremder Bauart und einen Außenkonus eines im Femur eingesetzten Prothesenschafts einer modularen Gelenkprothese, **dadurch gekennzeichnet, dass** die Vorrichtung einen Satz Prüfkörper (1) in Form von aufgeschnittenen Kugel- oder Zylindersegmenten (12) mit jeweils unterschiedlichen Innenkonen (3) umfasst, wobei eine Markierung (7) zum Feststellen des minimal zulässigen Abstand (AM) zwischen einer Stirnseite (9) des Außenkonus (10) des Schaftes (11) der Gelenkprothese und einem Dach (4) des Innenkonus (3) des Prüfkörpers nahe des Daches (4) auf einer Fläche (F) des Innenkonus (3) vorgesehen ist, und dass auf der Fläche (F) des Innenkonus (3) eine Längen-Messskala (8) zum Ermitteln der Einstecktiefe (ET) des Außenkonus (10) des Schaftes (11) in den Innenkonus (3) des jeweiligen Prüfkörpers (1) angeordnet ist.

2. Vorrichtung nach Anspruch 1, **dadurch gekennzeichnet, dass** die Innenkonen (3) des Prüfkörpers (1) des Satzes von Prüfkörpern verschiedene Winkel (α) und Durchmesser (D) aufweisen.

3. Vorrichtung nach Anspruch 1 oder 2 , **dadurch gekennzeichnet, dass** der minimal zulässige Abstand (AM) die Toleranzen der entsprechenden Durchmesser und Winkel des Innenkonus der Prüfkörper und des Außenkonus des Schaftes der Gelenkprothese berücksichtigt.

4. Vorrichtung nach Anspruch 1, **dadurch gekennzeichnet, dass** die zulässige Einstecktiefe (ET) des Außenkonus (10) des Schaftes (11) in den Innenkonus (3) des Prüfkörpers auf die Länge des Innenkonus(3) des Prüfkörpers abgestimmt ist.

5. Vorrichtung nach Anspruch 4, **dadurch gekenzennzeichnet**, dass die zulässige Einstecktiefe (ET) mindestens 7 mm beträgt.

6. Vorrichtung nach Anspruch 1, **dadurch gekennzeichnet, dass** dem Satz Prüfkörper (1) ein Satz Adapter zum Einsetzen bzw. Anpassen der Gelenkkugel zugeordnet ist.

7. Vorrichtung nach Anspruch 1, **dadurch gekennzeichnet, dass** das Zylindersegment (12) unterhalb seiner Öffnung (6) einen umlaufenden Bund (13) zum sicheren Aufstecken des Prüfkörpers (1) auf den Außenkonus (10) des Schaftes (11) aufweist.

## Claims

1. Device for determining the degree of fit of an adapter to be inserted into a provided for exchange joint ball of foreign construction and an external cone of an inserted into the femur prosthesis shaft of a modular joint prosthesis, **characterized in that** the device comprises a set of test pieces (1) in the shape of cut open sphere or cylinder segments (12) having different inner cones (3) respectively, wherein a mark (7) is provided for determining the minimal permissible distance (AM) between a front surface (9) of the external cone (10) of the shaft (11) of the joint prosthesis and a roof surface (4) of the inner cone (3) of the test piece near the roof surface (4) on a surface (F) of the inner cone (3), and that on the surface (F) of the inner cone (3) is provided a length measuring scale (8) for determining the insertion depth (ET) of the external cone (10) of the shaft (11) into the inner cone (3) of the respective test piece (1).

2. Device according to claim 1, **characterized in that** the inner cones (3) of the test pieces (1) of the set of test pieces have different angles (α) and diameters (D).

3. Device according to claim 1 or 2, **characterized in that** the minimal permissible distance (AM) allows for the tolerances of the respective diameters and angles of the inner cones of the test pieces and the external cone of the shaft of the joint prosthesis.

4. Device according to claim 1, **characterized in that** the permissible insertion depth (ET) of the external cone (10) of the shaft (11) into the inner cone (3) of the test piece is adjusted to the length of the inner cone (3) of the test piece.

5. Device according to claim 4, **characterized in that** the permissible insertion depth (ET) amounts to at least 7 mm.

6. Device according to claim 1, **characterized in that** the set of test pieces (1) is allocated a set of adapters for inserting or adjusting the joint ball.

7. Device according to claim 1, **characterized in that** the cylinder segment (12) below its opening (6) has a circumferential collar (13) for safely pushing the test piece (1) on the external cone (10) of the shaft (11).

## Revendications

1. Dispositif pour déterminer la compatibilité entre un adaptateur destiné à être inséré dans une rotule d'articulation de type étranger à remplacer et un cône extérieur d'une tige de prothèse insérée dans le fémur d'une prothèse articulaire modulaire, **caractérisé en ce que** le dispositif comprend un jeu de corps d'essai (1) sous forme de segments sphériques ou cylindriques (12) coupés ayant chacun des cônes intérieurs (3) différents, où un repère (7) permettant de déterminer la distance minimale autorisée (AM) entre une face frontale (9) du cône extérieur (10) de la tige (11) de la prothèse articulaire et un sommet (4) du cône intérieur (3) du corps d'essai est prévu à proximité du sommet (4) sur une surface (F) du cône intérieur (3), et **en ce qu'**une échelle de mesure de longueur (8) est disposée sur la surface (F) du cône intérieur (3), permettant de déterminer la profondeur d'insertion (ET) du cône extérieur (10) de la tige (11) dans le cône intérieur (3) du corps d'essai (1) respectif.

2. Dispositif selon la revendication 1, **caractérisé en ce que** les cônes intérieurs (3) du corps d'essai (1) du jeu de corps d'essai ont des angles (α) et diamètres (D) différents.

3. Dispositif selon la revendication 1 ou 2, **caractérisé en ce que** la distance minimale autorisée (AM) tient compte des tolérances des diamètres et angles correspondants du cône intérieur des corps d'essai et du cône extérieur de la tige de la prothèse articulaire.

4. Dispositif selon la revendication 1, **caractérisé en ce que** la profondeur d'insertion autorisée (ET) du cône extérieur (10) de la tige (11) dans le cône intérieur (3) du corps d'essai est réglée sur la longueur du cône intérieur (3) du corps d'essai.

5. Dispositif selon la revendication 4, **caractérisé en ce que** la profondeur d'insertion autorisée (ET) est d'au moins 7 mm.

6. Dispositif selon la revendication 1, **caractérisé en ce qu**'un jeu d'adaptateurs pour insérer ou adapter la rotule d'articulation est associé au jeu de corps d'essai (1).

7. Dispositif selon la revendication 1, **caractérisé en ce que** le segment cylindrique (12) comporte au-dessous de son ouverture (6) un rebord périphérique (13) permettant de placer le corps d'essai (1) en toute sécurité sur le cône extérieur (10) de la tige (11).
